# EUROPEAN PATENT SPECIFICATION

(11) **EP 1 666 445 B1**
(45) Date of publication and mention of the grant of the patent: **27.03.2019**
(21) Application number: 04787813.7
(22) Date of filing: 09.09.2004
(51) Int. Cl.: C07C 43/23, A23L 33/10

(54) **REDUCED COENZYME Q10 CRYSTAL EXCELLING IN STABILITY AND COMPOSITION CONTAINING REDUCED COENZYME Q10 CRYSTAL**
KRISTALL VON REDUZIERTEM COENZYM Q10 MIT EXZELLENTER STABILITÄT UND ZUSAMMENSETZUNG, DIE DEN KRISTALL VON REDUZIERTEM COENZYM Q10 ENTHÄLT
CRISTAL A COENZYME Q10 REDUIT DOTE D'UNE EXCELLENTE STABILITE ET COMPOSITION CONTENANT LEDIT CRISTAL

(30) Priority: 10.09.2003 JP 2003318335
(43) Date of publication of application: 07.06.2006
(73) Proprietor: KANEKA CORPORATION, Osaka (JP)
(72) Inventor: UEDA, Takahiro, Hyogo 655-0872 (JP); KITAMURA, Shiro, Hyogo 673-0882 (JP); KAWABE, Taizo, Hyogo 672-8044 (JP); KISHIDA, Hideyuki, Hyogo 675-0039 (JP); UEDA, Yasuyoshi, Hyogo 671-1227 (JP)
(74) Representative: Hoffmann Eitle
(86) International application number: PCT/JP2004/013150
(87) International publication number: WO 2005/033054

(56) References cited:
- EP-A1- 1 415 969
- JP-A- 2 212 421
- JP-A- 63 188 623
- JP-A- 2003 026 625
- JP-A- 2003 089 669
- JP-A- 2004 130 201
- DATABASE WPI Week 200362 Derwent Publications Ltd., London, GB; AN 2003-649294 XP002407208 & JP 2003 125734 A (NISSHIN PHARMA KK) 7 May 2003 (2003-05-07)

## Description

### TECHNICAL FIELD

The present invention relates to reduced coenzyme Q₁₀ crystals with excellent bioavailability and oxidation stability, and to a composition containing said reduced coenzyme Q₁₀ crystals. Reduced coenzyme Q₁₀ exhibits greater oral bioavailability than oxidized coenzyme Q₁₀, and is a compound that is useful as an ingredient in good foods, functional nutritive foods, specific health foods, nutritional supplements, nutrients, animal drugs, drinks, feeds, cosmetics, medicines, remedies, preventive drugs, etc.

### BACKGROUND ART

Coenzyme Q is an essential component of organisms in wide distribution from microorganisms to mammals, and is known as a mitochondrial electron transfer system constituent in cells of living bodies. Coenzyme Q serves as a transport of the electron transfer system, operating through cycles of oxidation and reduction within the mitochondria, and reduced coenzyme Q possesses antioxidant activity. The major component of coenzyme Q in humans is coenzyme Q₁₀, which is a coenzyme Q species having 10 repeating structures in its side chain, and the reduced form is usually present on the order of 40 to 90% in living bodies. The physiological activity of coenzyme Q involves the activation of energy production through mitochondrial activation, activation of cardiac function, stabilization of cell membranes, cell protection through antioxidant activity, and the like.

Oxidized coenzyme Q₁₀ is used as a health food in the United States and Europe, and as a medication for congestive heart failure in Japan. In recent years, it has come to be used in Japan as a functional nutritive food.

On the other hand, since reduced coenzyme Q itself has strong antioxidant action, it is possible to effectively increase the antioxidant activity in blood by supplying sufficient quantities of reduced coenzyme Q to blood. Increasing the antioxidant activity in blood is thought to have a wide range of usefulness for many diseases from being aggravated supposedly by active oxygen species, for example, preventing vascular lesions during ischemia-reperfusion, preventing restenosis in arteriosclerosis, preventing vascular lesions following cerebral infarction, preventing arteriosclerosis, preventing complications of diabetes.

It is known that reduced coenzyme Q₁₀ can be obtained, for example, by well-known conventional processes such as synthesis, fermentation, extraction from natural sources, and the like, and then concentrating the reduced coenzyme Q₁₀ fraction of the effluent resulting from chromatography (Japanese Kokai Publication Hei-10-109933). In this case, as described in the above-cited publication, the chromatographic concentration may be carried out after reduction of oxidized coenzyme Q₁₀ contained in the reduced coenzyme Q₁₀ with a conventional reducing agent such as sodium borohydride or sodium dithionite (sodium hydrosulfite), or reduced coenzyme Q₁₀ may be prepared by reacting an existing highly pure grade of coenzyme Q₁₀ with the reducing agent mentioned above.

Moreover, as a result of intensive research, the present inventors established processes for producing high-quality reduced coenzyme Q₁₀, which were disclosed in patent applications (WO 03/06408; WO 03/06409; WO 03/06410; WO 03/06411; WO 03/06412; WO 03/08363; and WO 03/32967).

The crystallization of coenzyme Q₁₀ has been described in the prior art. For example, JP 2003 125734 A describes a method or preparing crystallized coenzyme Q₁₀ which involves adding coenzyme Q₁₀ to an edible oil, heat dissolving and cooling to obtain precipitated coenzyme Q₁₀ in the form of crystals.

JP 2003 089669 A describes a method for producing reduced coenzyme Q₁₀. The method for crystallizing the reduced coenzyme Q₁₀ comprises adding a liquid phase containing the reduced coenzyme Q₁₀ in a high concentration to a poor solvent having a temperature lower than the solidification temperature of the reduced coenzyme Q₁₀ or the liquid phase.

However, reduced coenzyme Q₁₀ is readily oxidized to oxidized coenzyme Q₁₀ by a molecular oxygen. This oxidation is directly related to the problem of product quality such as formation of a difficult-to-remove byproduct, oxidized coenzyme Q₁₀, and immixture thereof into the product. In order to obtain high-purity reduced coenzyme Q₁₀ crystals and in order to stably maintain the obtained reduced coenzyme Q₁₀ crystals, it is important to suitably protect them from the above-mentioned oxidation.

### SUMMARY OF THE INVENTION

Accordingly, reduced coenzyme Q₁₀ crystals with improved stability, and a composition containing said reduced coenzyme Q₁₀ crystals, as well as a process for improving the stability of reduced coenzyme Q₁₀ have been desired.

As a result of intensive research carried out in view of the above-described state of affairs, the present inventors found that reduced coenzyme Q₁₀ crystals with excellent stability can be produced by crystallizing reduced coenzyme Q₁₀ in a fat and/or oil, and that when said crystals are analyzed by x-ray diffraction using a CuKα emission line, they possess characteristics that differ from ordinary reduced coenzyme Q₁₀ crystals that are crystallized from solvents, thereby achieving the present invention.

That is to say, the present invention provides a reduced coenzyme Q₁₀ crystal which comprises an intermediate intense peak at the diffraction angle (2θ) 3.1°, an intense peak at 20.2°, 23.0°, and a particularly intense peak at 18.7°, 19.0°, when analyzed with x-ray diffraction using a CuKα emission line. The reduced coenzyme Q₁₀ crystal also possesses the following characteristics (a) to (c) in x-ray diffraction using a CuKα emission line:
(a) If 100 is the peak intensity at 19.0°, the peak intensity at 3.1° is 25 or lower;
(b) If 100 is the peak intensity at 19.0°, the peak intensity at 18.7° is 80 or higher; and
(c) The intensity ratio of the peak at 20.2° and the peak at 23.0° is peak intensity at 20.2°/peak intensity at 23.0°, namely 0.7 or higher.

Furthermore, the present invention provides a composition containing a reduced coenzyme Q₁₀ crystal wherein said reduced coenzyme Q₁₀ crystal coexists with a fat and/or oil.

Moreover, the present invention provides a preparation for oral administration which contains said reduced coenzyme Q₁₀ crystal or the aforementioned composition.

In addition, the present invention provides a process for producing a reduced coenzyme Q₁₀ crystal which comprises adding reduced coenzyme Q₁₀ to a fat and/or oil and then heating to dissolve the reduced coenzyme Q₁₀ and/or adding heat-melted reduced coenzyme Q₁₀ to a heated fat and/or oil to prepare a mixture of reduced coenzyme Q₁₀ and a fat and/or oil, and cooling the mixture to crystallize reduced coenzyme Q₁₀.

Reduced coenzyme Q₁₀ crystals with improved stability can be produced in accordance with the present invention.

### DETAILED DESCRIPTION OF THE INVENTION

The present invention is described in detail below. In the present specification, when coenzyme Q₁₀ cited without specific indication, it refers to either the oxidized form or reduced form or, when both exist in admixture, a mixture of both forms.

The present invention reduced coenzyme Q₁₀ crystals can be produced by adding reduced coenzyme Q₁₀ to a fat and/or oil and then heating to dissolve the reduced coenzyme Q₁₀ and/or adding heat-melted reduced coenzyme Q₁₀ to a heated fat and/or oil to prepare a mixture of reduced coenzyme Q₁₀ and a fat and/or oil, and cooling the mixture to crystallize reduced coenzyme Q₁₀.

The reduced coenzyme Q₁₀ that is to be crystallized in the present invention can be produced by a well-known conventional process such as synthesis, fermentation, and extraction from natural sources. The reduced coenzyme Q₁₀ can be by itself, or can be mixed with oxidized coenzyme Q₁₀. Preferably, oxidized coenzyme Q₁₀ or a mixture of oxidized coenzyme Q₁₀ and reduced coenzyme Q₁₀, such as an existing high-purity coenzyme Q₁₀, can be reduced using a common reducing agent such as sodium hydrosulfite (sodium dithionite), sodium borohydride and ascorbic acid to obtain reduced coenzyme Q₁₀.

There are no particular limitations on the ratio of reduced coenzyme Q₁₀ in the total amount of coenzyme Q₁₀ (i.e., the total amount of reduced coenzyme Q₁₀ and oxidized coenzyme Q₁₀), but it is usually 20% by weight or more, preferably 40% by weight or more, more preferably 60% by weight or more, still more preferably 80% by weight or more, particularly preferably 90% by weight or more, and most preferably 96% by weight or more. The upper limit is 100% by weight and although there are no particular limitations, it is usually 99.9% by weight or less.

The fat and/or oil used can be a natural fat and/or oil derived from animals or plants, or a synthetic fat and/or oil or a processed fat and/or oil. Preferably, it is a species permitted for food or medicinal use. Examples of a vegetable fat and/or oil include coconut oil, palm oil, palm kernel oil, linseed oil, camellia oil, rice germ oil, rapeseed oil, rice oil, peanut oil, corn oil, wheat germ oil, soybean oil, perilla oil, cottonseed oil, sunflower seed oil, kapok oil, evening primrose oil, shea butter, sal fat, cacao butter, sesame oil, safflower oil, olive oil. Examples of an animal fat and/or oil include lard, milk fat, fish oil, beef tallow. Moreover, these can be processed to produce a fat and/or oil (e.g., hydrogenated oils) processed by fractionation, hydrogenation, ester exchange. Of course, medium-chain triglycerides (MCT), fatty acid partial glycerides, propylene glycol fatty acid esters, phospholipids can be used. Mixtures of these can also be used.

Examples of medium-chain triglycerides include triglycerides of fatty acids having 6 to 12 carbons each, and preferably 8 to 12 carbons each.

Examples of fatty acid partial glycerides include monoglycerides and diglycerides of fatty acids having 6 to 18 carbons each, and preferably 6 to 12 carbons each.

Examples of propylene glycol fatty acid esters include monoglycerides and diglycerides of fatty acids having 6 to 18 carbons each, and preferably 6 to 12 carbons each.

Examples of phospholipids include egg yolk lecithin, refined soybean lecithin, phosphatidylcholine, phosphatidylethanolamine, phosphatidylserine, sphingomyelin, dicetyl phosphoric acid, stearyl amine, phosphatidylglycerol, phosphatidic acid, phosphatidyl inositolamine, cardiolipin, ceramide phosphorylethanolamine, ceramide phosphorylglycerol, and mixtures thereof.

Of the above fats and/or oils, vegetable fats and/or oils, synthetic and processed fats and/or oils, and phospholipids are advantageous from the standpoint of ease of handling and odor. In selecting these fats and/or oils, consideration should be given to price, stability of reduced coenzyme Q₁₀, solubility of coenzyme Q₁₀, and the like. For example, coconut oil, palm oil, palm kernel oil, rapeseed oil, rice oil, soybean oil, cottonseed oil, olive oil, MCT, phospholipids are advantageous, and rice oil, soybean oil, rapeseed oil, MCT, phospholipids are particularly advantageous.

A fat and/or oil containing reduced coenzyme Q₁₀ before crystallization can be obtained by heating and melting existing reduced coenzyme Q₁₀ crystals at the melting point of reduced coenzyme Q₁₀ (49°C) or higher, and then adding to a fat and/or oil, or adding existing reduced coenzyme Q₁₀ crystals to a fat and/or oil, and then heating and dissolving, for example, heating to 50 to 90°C. Moreover, a solution of a fat and/or oil containing oxidized coenzyme Q₁₀ can be reduced using a reducing agent such as ascorbic acid, to produce a fat and/or oil containing reduced coenzyme Q₁₀.

There are no particular limitations on the heating/melting or heating/dissolving temperatures, but it is usually about 50°C or higher, preferably about 55°C or higher, more preferably about 60°C or higher, with the upper limit usually about 90°C or lower, preferably about 85°C or lower, and more preferably about 80°C or lower. It is usually carried out at about 50 to 90°C, preferably about 55 to 85°C, more preferably about 55 to 80°C, and still more preferably about 60 to 80°C.

Crystallization of reduced coenzyme Q₁₀ is carried out by cooling the above-mentioned fat and/or oil containing reduced coenzyme Q₁₀.

In addition to the above-mentioned fats and/or oils, other ingredients can be added as appropriate during the process of crystallization of reduced coenzyme Q₁₀. There are no particular limitations on these ingredients, and examples thereof include excipients, disintegrators, lubricants, binders, antioxidants, colorants, anticoagulation agents, absorption promoters, solubilizers for the active ingredients, stabilizers, viscosity modifier, and the like. Of course, the coexistence of active ingredients other than coenzyme Q₁₀ is not precluded, and the above ingredients can be added after crystallizing reduced coenzyme Q₁₀.

There are no particular limitations on the above-mentioned excipients, and examples thereof include white sugar, lactose, glucose, corn starch, mannitol, crystalline cellulose, calcium phosphate, calcium sulfate, and the like.

There are no particular limitations on the above-mentioned disintegrators, and examples thereof include starch, agar, calcium citrate, calcium carbonate, sodium hydrogencarbonate, dextrin, crystalline cellulose, carboxymethylcellulose, tragacanth, and the like.

There are no particular limitations on the above-mentioned lubricants, and examples thereof include talc, magnesium stearate, polyethylene glycol, silica, and the like.

There are no particular limitations on the above-mentioned binders, and examples thereof include ethyl cellulose, methyl cellulose, hydroxypropyl methylcellulose, tragacanth, shellac, gelatin, gum arabic, polyvinylpyrrolidone, polyvinylalcohol, polyacrylic acid, polymethacrylic acid, sorbitol, and the like.

There are no particular limitations on the above-mentioned antioxidants, and examples thereof include ascorbic acid, tocopherols, vitarmin A, beta-carotene, sodium hydrogensulfite, sodium thiosulfate, sodium pyrosulfite, citric acid, and the like.

There are no particular limitations on the above-mentioned colorants, and examples thereof include substances permitted for addition to pharmaceuticals and foods.

There are no particular limitations on the above-mentioned anticoagulation agents, and examples thereof include stearic acid, talc, light silicic acid anhydride, silicon dioxide hydride, and the like.

There are no particular limitations on the above-mentioned absorption promoters, and examples thereof include higher alcohols; higher fatty acids; surfactants such as sucrose fatty acid esters, sorbitan fatty acid esters, polyoxyethylene sorbitan fatty acid esters, polyglycerine fatty acid esters, and the like.

There are no particular limitations on the above-mentioned solubilizers for active ingredients, and examples thereof include fumaric acid, succinic acid, malic acid, and other organic acids, and the like.

There are no particular limitations on the above-mentioned stabilizers, and examples thereof include benzoic acid, sodium benzoate, ethyl parahydroxybenzoate, beeswax, and the like.

There are no particular limitations on the above-mentioned viscosity modifiers, and examples thereof include beeswax, and the like.

Examples of active components other than the coenzyme Q₁₀ include amino acids, vitamins, minerals, polyphenols, organic acids, saccharides, peptides, proteins, and the like.

There are no particular limitations on the crystallization temperature (cooling temperature during crystallization) of the reduced coenzyme Q₁₀, but from the standpoint of the crystallization rate, it is usually 30°C or less, preferably 25°C or less, more preferably 20°C or less, still more preferably 15°C or less, and most preferably 10°C or less. The lower limit is the solidification temperature of the system. In ordinary practice, 0°C to 25°C is suitable.

The concentration of reduced coenzyme Q₁₀ during crystallization is the weight of reduced coenzyme Q₁₀ with respect to the weight of a fat and/or oil used, and it is preferably 75% by weight or less, more preferably 50% by weight or less, still more preferably 30% by weight or less, and most preferably 20% by weight or less. The lower limit of the concentration is usually 0.1% by weight, preferably 1% by weight, and more preferably 3% by weight. Usually 0.1 to 75% by weight, preferably 1 to 50% by weight, and more preferably 3 to 30% by weight is suitable for actual practice.

The thus-obtained reduced coenzyme Q₁₀ crystals may be used after separation, or may be used as a composition containing reduced coenzyme Q₁₀ crystals as being mixed with a fat and/or oil.

When separating reduced coenzyme Q₁₀ crystals after crystallization, the concentration of reduced coenzyme Q₁₀ during crystallization is the weight of reduced coenzyme Q₁₀ with respect to the weight of the fat and/or oil used, and it is usually about 30% by weight or less, preferably about 20% by weight or less, more preferably about 15% by weight or less, particularly preferably about 12% by weight or less, and most preferably about 10% by weight or less. By maintaining this concentration, it becomes possible to carry out crystallization and separation of the crystals on an industrial scale. From the standpoint of productivity, the lower limit of the concentration is usually about 1% by weight, and preferably about 2% by weight. Usually, crystallization and separation can be preferably carried out at about 1 to 30% by weight.

When carrying out crystallization, it is advantageous to add seed crystals so as to prevent a formation of supersaturation, and to smoothly implement nucleation and crystal growth.

Moreover, it is also possible to carry out crystallization under forced flowing. For preventing the state of supersaturation from occurring and thereby allowing nucleation and crystal growth to proceed smoothly and from the viewpoint of obtaining high-quality products, the flowing is generally brought about by a power required for stirring per unit volume of not weaker than about 0.01 kW/m³, preferably not weaker than about 0.1 kW/m³, and more preferably not weaker than about 0.3 kW/m³. The forced flowing is generally provided by the turning of a stirring blade(s). However, the use of a stirring blade(s) is not always necessary if the above flowing can be otherwise obtained. For example, it is possible to utilize a method based on liquid circulation, and the like method.

In the process of crystallization, it is advantageous to control the crystallization weight per unit time and to control the formation of supersaturation. An advantageous crystallization weight per unit time is, for example, below a crystallization rate of about 50% of the total weight per unit time (i.e., maximum of 50% by weight/hour), and preferably below a crystallization rate of about 25% of the total crystal weight per unit time (i.e., maximum of 25% by weight/hour).

The cooling rate is usually about 40°C/hour or less, and preferably about 20°C/hour or less.

It is also allowable to separate and obtain reduced coenzyme Q₁₀ crystals after carrying out crystallization as above where necessary. In this case, for example, after crystallization, reduced coenzyme Q₁₀ crystals can be obtained as a wet product, by such a solid-liquid separation technique as centrifugation, pressure filtration, or vacuum filtration, if necessary followed by cake washing. Furthermore, they can be obtained also as a dry product by charging the wet product in a reduced pressure drier (vacuum drier) internally purged with an inert gas and drying the same under reduced pressure.

The present invention reduced coenzyme Q₁₀ crystals obtained as above showed different characteristics from the ordinary reduced coenzyme Q₁₀ crystals crystallized from solvents, when analyzed by X-ray diffraction using CuKα emission line. And these crystals exhibited outstanding stability, further showed excellent bioabsorbability.

When the reduced coenzyme Q₁₀ crystals of the present invention are analyzed by x-ray diffraction using a CuKα emission line, they comprise an intermediate intense peak at the diffraction angle (2θ) 3.1°, an intense peak at 20.2°, 23.0°, and a particularly intense peak at 18.7° and 19.0°, and they are distinguished from the ordinary coenzyme Q₁₀ crystals that are crystallized from solvents.

The reduced coenzyme Q₁₀ crystals possess a combination of all three of the following characteristics:
(a) If 100 is the peak intensity at 19.0°, the peak intensity at 3.1° is 25 or lower, and preferably 20 or lower.
(b) If 100 is the peak intensity at 19.0°, the peak intensity at 18.7° is 80 or higher, and preferably 85 or higher.
(c) The intensity ratio of the peak at 20.2° and the peak at 23.0° is peak intensity at 20.2°/peak intensity at 23.0°, namely 0.7 or higher, and preferably 0.75 or higher.

It should be noted that in indicating diffraction angles, errors could occur depending on the apparatus used, so an error on the order of ±0.2° is within the scope of the present invention.

Furthermore, in cases where the base line of the x-ray diffraction pattern is disturbed by the influence of amorphous residue in the crystals, and the like, it is advantageous to correct the base line by eliminating the interference of the amorphous matter.

After crystallizing reduced coenzyme Q₁₀, it can be used without further treatment as a composition wherein reduced coenzyme Q₁₀ crystals and a fat and/or oil are found together, or the coenzyme Q₁₀ crystals can be separated, and then used. The separated coenzyme Q₁₀ crystals can then be added to the desired fat and/or oil mentioned above, and the like, to produce a composition containing reduced coenzyme Q₁₀ crystals. Of course, in addition to coenzyme Q₁₀ crystals and a fat and/or oil, excipients, disintegrators, lubricants, binders, antioxidants, colorants, anticoagulation agents, absorption promoters, solubilizers for the active ingredients, stabilizers, viscosity modifiers, and active ingredients other than coenzyme Q₁₀, which are above mentioned, are not precluded from being added to said composition.

In the composition containing reduced coenzyme Q₁₀ crystals according to the present invention, the weight of reduced coenzyme Q₁₀ with respect to the weight of the fat and/or oil to be used is preferably about 75% by weight or less, more preferably about 50% by weight or less, still more preferably about 30% by weight or less, and particularly preferably about 20% by weight or less. The lower limit of concentration is usually about 0.1% by weight, preferably about 1% by weight, and more preferably about 3% by weight. Usually about 0.1 to 75% by weight, preferably about 1 to 50% by weight, and more preferably about 3 to 30% by weight is suitable for actual practice.

The present invention makes it possible to produce reduced coenzyme Q₁₀ crystals with excellent stability or a composition containing said reduced coenzyme Q₁₀ crystals. The thus-obtained crystals or composition containing said crystals are also excellent in bioabsorbability.

The resulting reduced coenzyme Q₁₀ crystals or composition containing said reduced coenzyme Q₁₀ crystals can be used as such, or can be further processed to produce capsules (hard capsules, soft capsules, or microcapsules), tablets, syrups, beverages, or other preparations for oral administration; or can be further processed to produce creams, suppositories, dentifrices, or other formulations. Preparations for oral administration are preferable, capsules are typically preferable, and soft capsules are particularly advantageous.

Of course, it is also within the scope of the present invention to form capsules containing said reduced coenzyme Q₁₀ crystals wherein a dissolved reduced coenzyme Q₁₀ in a fat and/or oil is formed into capsules and then cooled to be crystallized out as reduced coenzyme Q₁₀ crystals, said dissolved reduced coenzyme Q₁₀ in a fat and/or oil being a mixture of reduced coenzyme Q₁₀ and a fat and/or oil which is produced by adding reduced coenzyme Q₁₀ to a fat and/or oil and then heating to dissolve the reduced coenzyme Q₁₀ and/or by adding heat-melted reduced coenzyme Q₁₀ to a heated fat and/or oil.

There are no particular limitations on the capsule base material used for preparing capsules, and examples thereof include gelatin derived from cow bones, cowhide, pig skin, fish skin, and the like, but other base materials (substances which can be used as food additives, for example, thickeners and stabilizers derived from seaweed such as carrageenan and alginic acid, derived from plant seeds such as locust bean gum and guar gum; manufacturing agents containing celluloses, and the like) can also be used.

Furthermore, the present invention reduced coenzyme Q₁₀ crystals or compositions containing said reduced coenzyme Q₁₀ crystals, as well as the above-mentioned capsules, can be used, when appropriate, as an additive in the preparation of bread, pasta, Japanese zosui (porridge of rice and vegetables), boiled rice dishes, cakes, confectioneries, and the like. Of course, use in the preparation of other foods is not precluded.

The protection effect from oxidation of the present invention reduced coenzyme Q₁₀ crystals or compositions containing said reduced coenzyme Q₁₀ crystals can be enhanced by the manufacture and storage thereof in a deoxygenated atmosphere. A deoxygenated atmosphere can be achieved by replacement with an inert gas, pressure reduction, boiling, or combinations of these. At least, replacement with an inert gas, that is, the use of an inert gas environment, is suitable. Examples of said inert gas include nitrogen gas, helium gas, argon gas, hydrogen gas, carbonic dioxide, or the like, and nitrogen gas is preferable.

In said reduced coenzyme Q₁₀ crystals and compositions and preparations for oral administration containing said reduced coenzyme Q₁₀ crystals, it can be expected that after storage for a specified period of time, the reduced coenzyme Q₁₀ would be maintained in a weight ratio [reduced coenzyme Q₁₀/(reduced coenzyme Q₁₀ + oxidized coenzyme Q₁₀)] of 90% by weight or more, and preferably 95% by weight or more. Said period of storage is, for example, 1 day or longer, preferably 1 week or longer, more preferably 1 month or longer, still more preferably a half year or longer, particularly preferably 1 year or longer, and most preferably 2 years or longer.

In accordance with the present invention, it is possible to easily produce reduced coenzyme Q₁₀ crystals with excellent stability or a composition containing said reduced coenzyme Q₁₀ crystals. Moreover, said reduced coenzyme Q₁₀ crystals and composition containing said crystals are excellent in bioabsorbability, and applied in wide range of fields since they are suited for such use as compositions for foods and medical use or preparations for oral administration, thus has great advantage.

### BRIEF DESCRIPTION OF THE DRAWINGS

FIG. 1 is an x-ray diffraction pattern (using a CuKα emission line) of reduced coenzyme Q₁₀ crystals obtained by dissolving reduced coenzyme Q₁₀ in rapeseed oil and cooling.
FIG. 2 is an x-ray diffraction pattern (using a CuKα emission line) of reduced coenzyme Q₁₀ crystals obtained by dissolving reduced coenzyme Q₁₀ in 6% hydrated ethanol and cooling.

### BEST MODE FOR CARRYING OUT THE INVENTION

The present invention is described in further detail below with examples, but the present invention is not limited to these examples only. Furthermore, the purity of the reduced coenzyme Q₁₀ and the weight ratio of reduced coenzyme Q₁₀/oxidized coenzyme Q₁₀ in the examples are determined by HPLC analysis as below, but a standard for the purity of the resulting reduced coenzyme Q₁₀ is not established on the basis of the limiting values for purity in the present invention, and likewise, the weight ratios of reduced coenzyme Q₁₀/oxidized coenzyme Q₁₀ are not an upper limit for establishing a standard.

### (Conditions for HPLC Analysis)

Column: SYMMETRY C18 (Waters product),
250 mm (length), 4.6 mm (inner diameter)
Mobile phase: Ethanol/methanol = 4/3(v/v)
Detection wavelength: 210 nm
Flow rate: 1 mL/min
Reduced coenzyme Q₁₀ retention time: 9.1 min
Oxidized coenzyme Q₁₀ retention time: 13.3 min

In addition, an x-ray diffraction method using CuKα-emission line in Examples was carried out as follows.
Apparatus: rotational anticathode type x-ray diffraction apparatus Geiger-Flex RAD-rA [Rigaku Corporation product]
X-ray: CuKα emission line
X-ray intensity: 40kV, 100 mA
Angle area: 2θ = 2 to 60°
Scanning rate: 2°/min.
Sampling interval: 0.02 seconds
Divergence slit: 1°
Receiving slit: 0.60°
Scatter slit: 1°

### (Reference Example 1)

To 1,000 g of ethanol was added 100 g of oxidized coenzyme Q₁₀ and 60 g of L-ascorbic acid. These were stirred at 78°C, and a reduction reaction was carried out. After 30 hours, it was cooled to 50°C, and 400 g of ethanol and 100 g of water were added, while maintaining the same temperature. While stirring this ethanol solution (power required for stirring per unit volume: 0.3 kW/m³), it was cooled to 2°C at a cooling rate of 10°C/hour, resulting in a white slurry. The resulting slurry was then vacuum filtered, and the wet crystals were washed successively with cold ethanol, cold water, and cold ethanol, and moreover, the wet crystals were vacuum dried (20 to 35°C, 133 to 4000 Pa (1 to 30 mmHg)) to produce 95 g of dry white crystals (yield as appearance 95 mol%). With the exception of vacuum drying, all operations were carried out in a nitrogen atmosphere. The weight ratio of reduced coenzyme Q₁₀/oxidized coenzyme Q₁₀ in the resulting crystals was 99.5/0.5.

### (Example 1)

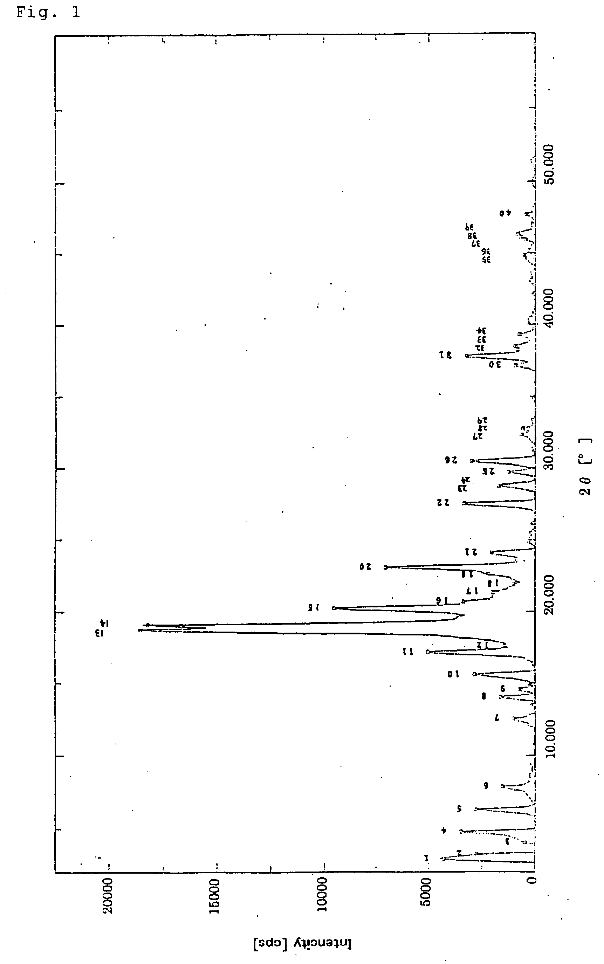

10 g of the reduced coenzyme Q₁₀ obtained in Reference Example 1 (weight ratio of reduced coenzyme Q₁₀/oxidized coenzyme Q₁₀ was 99.5/0.5) was added to 190 g of rapeseed oil at 25°C, and while stirring at 60°C (power required for stirring per unit volume: 0.3 kW/m³), the reduced coenzyme Q₁₀ was completely dissolved. While further stirring (power required for stirring per unit volume: 0.3 kW/m³), it was cooled to 5°C at a cooling rate of 10°C/hour, and crystals were crystallized out. All of the above operations were carried out in a nitrogen atmosphere. The resulting slurry was vacuum filtered, and thoroughly washed with hexane, after which the wet crystals were vacuum dried (20 to 35°C, 133 to 4000 Pa (1 to 30 mmHg)), resulting in 8.7 g of reduced coenzyme Q₁₀ crystals, as shown in the x-ray diffraction pattern of FIG. 1, in which a CuKα emission line is used. As shown in the above x-ray diffraction pattern, these crystals comprised intermediate intense peaks at the diffraction angle (2θ) 3.1°, intense peaks at 20.2°, 23.0°, and particularly intense peaks at 18.7°, 19.0°. The weight ratio of reduced coenzyme Q₁₀/oxidized coenzyme Q₁₀ in the resulting crystals was 99.3/0.7.

### (Example 2)

10 g of the reduced coenzyme Q₁₀ obtained in Reference Example 1 (weight ratio of reduced coenzyme Q₁₀/oxidized coenzyme Q₁₀ was 99.5/0.5) was added to 190 g of a medium-chain triglyceride (MCT, C8:C10 = 6:4), at 25°C, and while stirring at 60°C (power required for stirring per unit volume: 0.3 kW/m³), the reduced coenzyme Q₁₀ was completely dissolved. While further stirring (power required for stirring per unit volume: 0.3 kW/m³), it was cooled to 5°C at a cooling rate of 10°C/hour, and crystals were precipitated out. All of the above operations were carried out in a nitrogen atmosphere. The resulting slurry was vacuum filtered, and thoroughly washed with hexane, after which the wet crystals were vacuum dried (20 to 35°C, 133 to 4000 Pa (1 to 30 mmHg)), resulting in 6.5 g of reduced coenzyme Q₁₀ crystals. The weight ratio of reduced coenzyme Q₁₀/oxidized coenzyme Q₁₀ in the resulting crystals was 99.3/0.7.

### (Comparative Example 1)

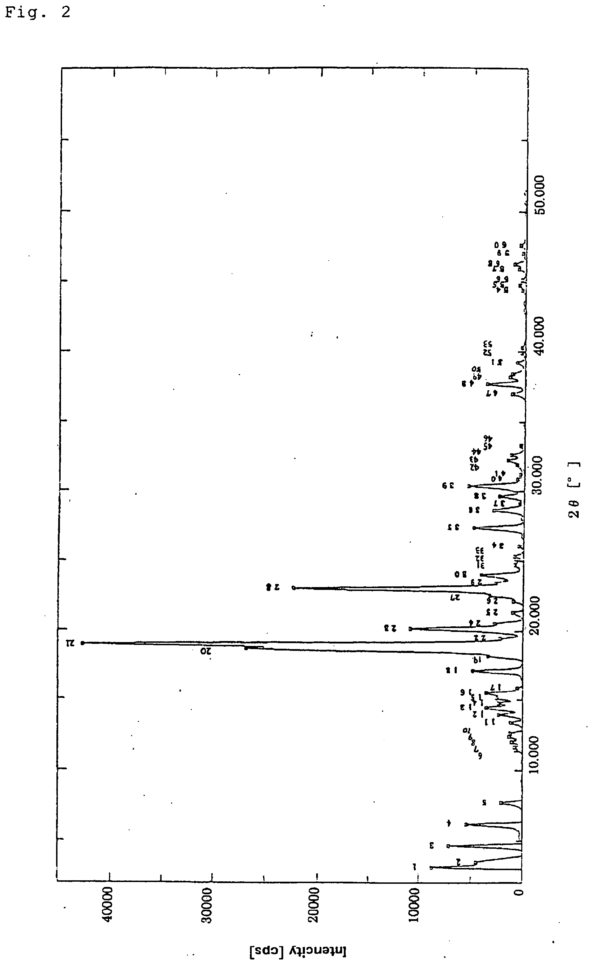

10 g of the reduced coenzyme Q₁₀ obtained in Reference Example 1 (weight ratio of reduced coenzyme Q₁₀/oxidized coenzyme Q₁₀ was 99.5/0.5) was added to 150 g of 6% hydrated ethanol at 25°C, and while stirring at 60°C, the reduced coenzyme Q₁₀ was completely dissolved. While further stirring (power required for stirring per unit volume: 0.3 kW/m³), it was cooled to 5°C at a cooling rate of 10°C/hour, and crystals were precipitated out. All of the above operations were carried out in a nitrogen atmosphere. The resulting slurry was vacuum filtered, and thoroughly washed successively with ethanol, water, and ethanol, after which the wet crystals were vacuum dried (20 to 35°C, 133 to 4000 Pa (1 to 30 mmHg)), resulting in 9.6 g of reduced coenzyme Q₁₀ crystals, as shown in the x-ray diffraction pattern of FIG. 2, in which a CuKα emission line is used. The weight ratio of reduced coenzyme Q₁₀/oxidized coenzyme Q₁₀ in the resulting crystals was 99.3/0.7.

### (Example 3)

The reduced coenzyme Q₁₀ crystals obtained in Example 1, Example 2, and Comparative Example 1 were stored for 4 days in air at 30°C, and shielded from light. After 4 days, the weight ratio of reduced coenzyme Q₁₀/oxidized coenzyme Q₁₀ was measured. The results are given in Table 1.

**Table 1**

| | R |
|---|---|
| Crystals obtained in Example 1 | 96.6/3.4 |
| Crystals obtained in Example 2 | 90.5/9.5 |
| Crystals obtained in Comparative Example 1 | 79.0/21.0 |

| | |
|---|---|
| R: Weight ratio of reduced coenzyme Q₁₀/ oxidized coenzyme Q₁₀ | |

From these results, it is found that the present invention reduced coenzyme Q₁₀ crystals obtained by crystallizing reduced coenzyme Q₁₀ from a fat and/or oil are excellent in stability as compared with ordinary reduced coenzyme Q₁₀ crystals that are crystallized from solvents.

### (Reference Example 2)

Using the reduced coenzyme Q₁₀ obtained in Example 1 and the reduced coenzyme Q₁₀ obtained in Comparative Example 1, the blood concentrations of reduced coenzyme Q₁₀ were measured when they were orally administered to rats. Each samples and rapeseed oil were added to aqueous solution of 0.2 % Tween 80 to prepare suspension in which reduced coenzyme Q₁₀ : rapeseed oil = 1 : 2. The suspension was orally administered to SD rats (male, 6 rats in one group) forcibly using a stomach sonde in 30 mg (reduced coenzyme Q₁₀) / kg (rat body weight) . Thereafter, the blood was sampled with time, and the plasma concentration of reduced coenzyme Q₁₀ was measured by HPLC.

The measurement results of the plasma concentrations of reduced coenzyme Q₁₀ are shown in Table 2.

**Table 2**

| | Cₘₐₓ(mg/ml) | Tₘₐₓ(hr) | AUC (0-24) (mg/ml·hr) |
|---|---|---|---|
| Reduced coenzyme Q₁₀ crystals obtained in Example 1 | 1.81±0.81 | 4 | 25.04±6.32 |
| Reduced coenzym e Q₁₀ crystals obtained in Com parative Exam p le 1 | 1.35±0.27 | 8 | 21.77±4.08 |

| | | | |
|---|---|---|---|
| (Mean±S.D.) Cₘₐₓ(mg/ml) ···Maximum plasm a concentration Tₘₐₓ (hr)···Time to maximum plasm a concentration AUC (0-24) (mg/ml·hr) ···Area under the plasm a concentration time curve | | | |

When the reduced coenzyme Q₁₀ crystals obtained in Example 1 and the reduced coenzyme Q₁₀ crystals obtained in Comparative Example 1 were administered to animals, and the plasma concentrations of coenzyme Q₁₀ were determined and compared, bioavailability of reduced coenzyme Q₁₀ of the present invention is clearly higher than those obtained by crystallization from solvents as shown in Table 2. Accordingly, it was made clear that significantly high bioavailability can be obtained when reduced coenzyme Q₁₀ of the present invention is used.

### (Example 4)

160 g of rapeseed oil, 10 g of a hydrogenated oil, 5 g of lecithin, and 5 g of beeswax were mixed at 70°C. At the same temperature, 20 g of the reduced coenzyme Q₁₀ obtained in Reference Example 1 (weight ratio of reduced coenzyme Q₁₀/oxidized coenzyme Q₁₀ was 99.5/0.5) was added, and while stirring (power required for stirring per unit volume: 0.3 kW/m³), the reduced coenzyme Q₁₀ was completely dissolved. After dissolution, the solution was cooled to 5°C at a rate of 10°C hour while maintaining the above stirring, and crystals were precipitated out. All of the above operations were carried out in a nitrogen atmosphere. After returning the temperature of the composition containing the reduced coenzyme Q₁₀ crystals to 20°C, a soft gelatin capsule preparation was produced by a conventional manner with 300 mg of the composition containing reduced coenzyme Q₁₀ crystals (corresponding to 30 mg of reduced coenzyme Q₁₀) per capsule.

### (Example 5)

160 g of rapeseed oil, 10 g of a hydrogenated oil, 5 g of lecithin, and 5 g of beeswax were mixed at 70°C. At the same temperature, 20 g of the reduced coenzyme Q₁₀ obtained in Reference Example 1 (weight ratio of reduced coenzyme Q₁₀/oxidized coenzyme Q₁₀ was 99.5/0.5) was added, and while stirring (power required for stirring per unit volume: 0.3 kW/m³), the reduced coenzyme Q₁₀ was completely dissolved. All of the above operations were carried out in a nitrogen atmosphere. After cooling the composition containing the reduced coenzyme Q₁₀ crystals to 20°C, a soft gelatin capsule preparation was produced by a conventional manner with 300 mg of the composition containing reduced coenzyme Q₁₀ crystals (corresponding to 30 mg of reduced coenzyme Q₁₀) per capsule. When this soft gelatin capsule preparation was let stand for 24 hours at 5°C, reduced coenzyme Q₁₀ crystals precipitated out inside the soft gel capsules.

### INDUSTRIAL APPLICABILITY

In accordance with the present invention, it is possible to easily produce reduced coenzyme Q₁₀ crystals with excellent stability or a composition containing said reduced coenzyme Q₁₀ crystals. Moreover, said reduced coenzyme Q₁₀ crystals and composition containing said crystals are excellent in bioabsorbability, and applied in wide range of fields since they are suited for such use as compositions for foods and medical use or preparations for oral administration, thus has great advantage.

## Claims

1. A reduced coenzyme Q₁₀ crystal
which comprises an intermediate intense peak at the diffraction angle (2θ) 3.1°, an intense peak at 20.2°, 23.0°, and a particularly intense peak at 18.7°, 19.0°, when analyzed with x-ray diffraction using a CuKα emission line, wherein the crystal possesses the following characteristics (a) to (c) in x-ray diffraction using a CuKα emission line:
(a) If 100 is the peak intensity at 19.0°, the peak intensity at 3.1° is 25 or lower;
(b) If 100 is the peak intensity at 19.0°, the peak intensity at 18.7° is 80 or higher; and
(c) The intensity ratio of the peak at 20.2° and the peak at 23.0° is peak intensity at 20.2°/peak intensity at 23.0°, namely 0.7 or higher.

2. A composition containing a reduced coenzyme Q₁₀ crystal,
wherein the reduced coenzyme Q₁₀ crystal according to Claim 1 coexists with a fat and/or oil.

3. The composition according to Claim 2,
which is obtainable by adding reduced coenzyme Q₁₀ to a fat and/or oil and then heating to dissolve the reduced coenzyme Q₁₀ and/or adding heat-melted reduced coenzyme Q₁₀ to a heated fat and/or oil to prepare a mixture of reduced coenzyme Q₁₀ and a fat and/or oil, and cooling the mixture.

4. The composition according to Claim 2,
which is obtainable by adding the reduced coenzyme Q₁₀ crystal according to Claim 1 to a fat and/or oil.

5. The composition according to any one of Claims 2 to 4,
wherein the weight ratio of reduced coenzyme Q₁₀ to the weight of a fat and/or oil is 0.1 to 75% by weight.

6. The composition according to any one of Claims 2 to 5,
wherein the fat and/or oil is at least one species selected from coconut oil, palm oil, palm kernel oil, linseed oil, camellia oil, rice germ oil, rapeseed oil, rice oil, peanut oil, corn oil, wheat germ oil, soybean oil, perilla oil, cottonseed oil, sunflower seed oil, kapok oil, evening primrose oil, shea butter, sal fat, cacao butter, sesame oil, safflower oil, olive oil, lard, milk fat, fish oil, beef tallow, these fats and/or oils processed by fractionation, hydrogenation and/or ester exchange, medium-chain triglycerides, fatty acid partial glycerides, propylene glycol fatty acid esters, and phospholipids.

7. A preparation for oral administration,
which contains the reduced coenzyme Q₁₀ crystal according to Claim 1 or the composition according to any one of Claims 2 to 6.

8. The preparation for oral administration according to Claim 7,
which is in capsule form.

9. A process for producing a reduced coenzyme Q₁₀ crystal
which comprises adding reduced coenzyme Q₁₀ to a fat and/or oil and then heating to dissolve the reduced coenzyme Q₁₀ and/or adding heat-melted reduced coenzyme Q₁₀ to a heated fat and/or oil to prepare a mixture of reduced coenzyme Q₁₀ and a fat and/or oil, and cooling the mixture to crystallize reduced coenzyme Q₁₀.

10. The process according to Claim 9,
wherein the ratio of the weight of reduced coenzyme Q₁₀ to the weight of a fat and/or oil during crystallization is 0.1 to 75% by weight.

11. The process according to Claim 9 or 10,
wherein the fat and/or oil is at least one species selected from coconut oil, palm oil, palm kernel oil, linseed oil, camellia oil, rice germ oil, rapeseed oil, rice oil, peanut oil, corn oil, wheat germ oil, soybean oil, perilla oil, cottonseed oil, sunflower seed oil, kapok oil, evening primrose oil, shea butter, sal fat, cacao butter, sesame oil, safflower oil, olive oil, lard, milk fat, fish oil, beef tallow, these fats and/or oils processed by fractionation, hydrogenation and/or ester exchange, medium-chain triglycerides, fatty acid partial glycerides, propylene glycol fatty acid esters, and phospholipids.

12. The process according to any one of Claims 9 to 11,
wherein the reduced coenzyme Q₁₀ crystal is produced in a deoxygenated atmosphere.

## Patentansprüche

1. Kristall aus reduziertem Coenzym Q₁₀,
der einen mittelmäßig intensiven Peak bei einem Beugungswinkel (2θ) von 3,1°, einen intensiven Peak bei 20,2°, 23,0° und einen besonders intensiven Peak bei 18,7°, 19,0° umfasst, wenn er mit Röntgenbeugung unter Verwendung einer CuKα-Emissionslinie analysiert wird, wobei der Kristall die folgenden Eigenschaften (a) bis (c) in der Röntgenbeugung unter Verwendung einer CuKα-Emissionslinie aufweist:
(a) wenn 100 die Peakintensität bei 19,0° ist, ist die Peakintensität bei 3,1° 25 oder niedriger;
(b) wenn 100 die Peakintensität bei 19,0° ist, ist die Peakintensität bei 18,7° 80 oder höher; und
(c) das Intensitätsverhältnis des Peaks bei 20,2° und des Peaks bei 23,0° ist Peakintensität bei 20,2°/Peakintensität bei 23,0° nämlich 0,7 oder höher.

2. Zusammensetzung, die einen Kristall aus reduziertem Coenzym Q₁₀ enthält,
wobei der Kristall aus reduziertem Coenzym Q₁₀ gemäß Anspruch 1 neben einem Fett und/oder Öl vorhanden ist.

3. Zusammensetzung gemäß Anspruch 2,
die durch Zugeben von reduziertem Coenzym Q₁₀ zu einem Fett und/oder Öl und anschließendes Erwärmen zum Lösen des reduzierten Coenzym Q₁₀ und/oder Zugeben von durch Wärme geschmolzenem reduzierten Coenzym Q₁₀ zu einem erwärmten Fett und/oder Öl zur Herstellung einer Mischung aus reduziertem Coenzym Q₁₀ und einem Fett und/oder Öl und Abkühlen der Mischung erhältlich ist.

4. Zusammensetzung gemäß Anspruch 2,
die durch Zugeben des Kristalls aus reduziertem Coenzym Q₁₀ gemäß Anspruch 1 zu einem Fett und/oder Öl erhältlich ist.

5. Zusammensetzung gemäß irgendeinem der Ansprüche 2 bis 4,
wobei das Gewichtsverhältnis von reduziertem Coenzym Q₁₀ zu dem Gewicht eines Fetts und/oder Öls 0,1 bis 75 Gew.-% beträgt.

6. Zusammensetzung gemäß irgendeinem der Ansprüche 2 bis 5,
wobei das Fett und/oder Öl mindestens eine Art, ausgewählt aus Kokosnussöl, Palmöl, Palmkernöl, Leinsamenöl, Kamelienöl, Reiskernöl, Rapsöl, Reisöl, Erdnussöl, Maisöl, Weizenkeimöl, Sojaöl, Perillaöl, Baumwollsamenöl, Sonnenblumenkernöl, Kapoköl, Nachtkerzenöl, Sheabutter, Salfett, Kakaobutter, Sesamöl, Safloröl, Olivenöl, Schmalz, Milchfett, Fischöl, Rindertalg, diesen Fetten und/oder Ölen, die durch Fraktionierung, Hydrierung und/oder Esteraustausch verarbeitet wurden, mittelkettigen Triglyceriden, partiellen Fettsäureglyceriden, Propylenglykolfettsäureestern und Phospholipiden, ist.

7. Zubereitung zur oralen Verabreichung,
die den Kristall aus reduziertem Coenzym Q₁₀ gemäß Anspruch 1 oder die Zusammensetzung gemäß irgendeinem der Ansprüche 2 bis 6 enthält.

8. Zubereitung zur oralen Verabreichung gemäß Anspruch 7,
die in Kapselform vorliegt.

9. Verfahren zur Herstellung eines Kristalls aus reduziertem Coenzym Q₁₀,
das das Zugeben von reduziertem Coenzym Q₁₀ zu einem Fett und/oder Öl und das anschließende Erwärmen zum Lösen des reduziertem Coenzyms Q₁₀ und/oder das Zugeben von durch Wärme geschmolzenem reduzierten Coenzym Q₁₀ zu einem erwärmten Fett und/oder Öl zur Herstellung einer Mischung aus reduziertem Coenzym Q₁₀ und einem Fett und/oder Öl und das Abkühlen der Mischung zum Kristallisieren des reduzierten Coenzyms Q₁₀ umfasst.

10. Verfahren gemäß Anspruch 9,
wobei das Verhältnis des Gewichts des reduzierten Coenzyms Q₁₀ zu dem Gewicht eines Fetts und/oder Öls während der Kristallisation 0,1 bis 75 Gew.-% beträgt.

11. Verfahren gemäß Anspruch 9 oder 10,
wobei das Fett und/oder Öl mindestens eine Art, ausgewählt aus Kokosnussöl, Palmöl, Palmkernöl, Leinsamenöl, Kamelienöl, Reiskernöl, Rapsöl, Reisöl, Erdnussöl, Maisöl, Weizenkeimöl, Sojaöl, Perillaöl, Baumwollsamenöl, Sonnenblumenkernöl, Kapoköl, Nachtkerzenöl, Sheabutter, Salfett, Kakaobutter, Sesamöl, Safloröl, Olivenöl, Schmalz, Milchfett, Fischöl, Rindertalg, diesen Fetten und/oder Ölen, die durch Fraktionierung, Hydrierung und/oder Esteraustausch verarbeitet wurden, mittelkettigen Triglyceriden, partiellen Fettsäureglyceriden, Propylenglykolfettsäureestern und Phospholipiden, ist.

12. Verfahren gemäß irgendeinem der Ansprüche 9 bis 11,
wobei der Kristall aus reduziertem Coenzym Q₁₀ unter deoxygenierter Atmosphäre hergestellt wird.

## Revendications

1. Cristal de coenzyme Q10 réduite
qui comprend un pic d'intensité intermédiaire à l'angle de diffraction (2θ) 3.1°, un pic d'intensité à 20.2°, 23.0°, et un pic de particulière intensité à 18.7°, 19.0°, lorsqu'analysé par diffraction aux rayons X en utilisant une ligne d'émission CuKα :
(a) si 100 est l'intensité de pic à 19.0°, l'intensité de pic à 3.1° est de 25 ou inférieure ;
(b) si 100 est l'intensité de pic à 19.0°, l'intensité de pic à 18.7° est de 80 ou supérieure ; et
(c) le rapport d'intensité du pic à 20.2° et du pic à 23.0° est intensité de pic à 20.2°/intensité de pic à 23.0°, c'est-à-dire 0.7 ou plus.

2. Composition contenant un cristal de coenzyme Q10 réduite,
dans laquelle le cristal de coenzyme Q10 réduite selon la revendication 1 coexiste avec une graisse et/ou une huile.

3. La composition selon la revendication 2,
qui peut être obtenue par ajout de la coenzyme Q10 réduite à une graisse et/ou une huile et ensuite chauffage pour dissoudre la coenzyme Q10 réduite et/ou ajout de coenzyme Q10 réduite fondue à chaud à une huile et/ou une graisse chauffée pour préparer un mélange de coenzyme Q10 réduite et d'une graisse et/ou d'une huile, et refroidissement du mélange.

4. La composition selon la revendication 2,
qui peut être obtenue par ajout de la coenzyme Q10 réduite selon la revendication 1 à une graisse et/ou une huile.

5. La composition selon l'une quelconque des revendications 2 à 4,
dans laquelle le rapport en poids de la coenzyme Q10 réduite au poids d'une graisse et/ou d'une huile est de 0,1 à 75% en poids.

6. La composition selon l'une quelconque des revendications 2 à 5,
dans laquelle la graisse et/ou l'huile est au moins un espèce choisie parmi l'huile de noix de coco, l'huile de palme, l'huile de noyaux de palme, l'huile de lin, l'huile de camellia, l'huile de germes de riz, l'huile de colza, l'huile de riz, l'huile de cacahuète, l'huile de maïs, l'huile de germes de blé, l'huile de soja, l'huile de périlla, l'huile de graines de coton, l'huile de graines de tournesol, l'huile de kapok, l'huile de primevères, le beurre de karité, la graisse de sal, le beurre de cacao, l'huile de sésame, l'huile de carthame, l'huile d'olive, le lard, la graisse de lait, l'huile de poisson, le suif de boeuf, ces graisses et/ou huiles étant transformées par fractionnement, hydrogénation et/ou échange d'ester, des triglycérides à chaîne moyenne, des glycérides partiels d'acide gras, des esters d'acide gras et de propylène glycol, et des phospholipides.

7. Préparation pour l'administration orale,
qui contient le cristal de coenzyme Q10 réduite selon la revendication 1 ou la composition selon l'une quelconque des revendications 2 à 6.

8. La préparation pour l'administration orale selon la revendication 7,
qui est sous forme de capsules.

9. Procédé pour la production d'un cristal de coenzyme Q10 réduite,
qui comprend l'ajout de la coenzyme Q10 réduite à une graisse et/ou une huile et ensuite le chauffage pour dissoudre la coenzyme Q10 réduite et/ou l'ajout de coenzyme Q10 réduite fondue à chaud à une huile et/ou une graisse chauffée pour préparer un mélange de coenzyme Q10 réduite et d'une graisse et/ou d'une huile, et le refroidissement du mélange pour cristalliser la coenzyme Q10 réduite.

10. Le procédé selon la revendication 9,
dans lequel le rapport du poids de la coenzyme Q10 réduite au poids d'une graisse et/ou d'une huile pendant la cristallisation est de 0,1 à 75% en poids.

11. Le procédé selon la revendication 9 ou 10,
dans lequel la graisse et/ou l'huile est au moins un espèce choisie parmi l'huile de noix de coco, l'huile de palme, l'huile de noyaux de palme, l'huile de lin, l'huile de camellia, l'huile de germes de riz, l'huile de colza, l'huile de riz, l'huile de cacahuète, l'huile de maïs, l'huile de germes de blé, l'huile de soja, l'huile de périlla, l'huile de graines de coton, l'huile de graines de tournesol, l'huile de kapok, l'huile de primevères, le beurre de karité, la graisse de sal, le beurre de cacao, l'huile de sésame, l'huile de carthame, l'huile d'olive, le lard, la graisse de lait, l'huile de poisson, le suif de boeuf, ces graisses et/ou huiles étant transformées par fractionnement, hydrogénation et/ou échange d'ester, des triglycérides à chaîne moyenne, des glycérides partiels d'acide gras, des esters d'acide gras et de propylène glycol, et des phospholipides.

12. Le procédé selon l'une quelconque des revendications 9 à 11,
dans lequel le cristal de coenzyme Q10 réduite est produit dans un atmosphère désoxygénée.
